(19) Europäisches Patentamt
European Patent Office
Office européen des brevets

(11) Veröffentlichungsnummer: **0 185 961**
**A2**

(12) **EUROPÄISCHE PATENTANMELDUNG**

(21) Anmeldenummer: **85115155.5**

(22) Anmeldetag: **29.11.85**

(51) Int. Cl.[4]: **C 07 D 233/90**
C 07 D 233/64, C 07 D 403/04
C 07 D 403/06, C 07 D 403/12
C 07 D 405/04, C 07 D 409/04
C 07 D 411/04, C 07 D 413/04
C 07 D 413/06, C 07 D 417/04

(30) Priorität: **08.12.84 DE 3444918**

(43) Veröffentlichungstag der Anmeldung:
**02.07.86 Patentblatt 86/27**

(84) Benannte Vertragsstaaten:
**AT BE CH DE FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Schmierer, Roland, Dr.**
**An der Weinleite 5A**
**D-8901 Todtenweis(DE)**

(72) Erfinder: **Mildenberger, Hilmar, Dr.**
**Fasanenstrasse 24**
**D-6233 Kelkheim (Taunus)(DE)**

(72) Erfinder: **Handte, Reinhard, Dr.**
**Theilweg 23**
**D-8901 Gablingen(DE)**

(72) Erfinder: **Bürstell, Helmut, Dr.**
**Am Hohlacker 65**
**D-6000 Frankfurt am Main 50(DE)**

(54) **1-Phenyl-imidazol-5-carbonsäure-Derivate, ein Verfahren zu ihrer Herstellung und ihre Verwendung als Wachstumsregulatoren.**

(57) Die neuen Derivate von im Phenylring substituierten 1-Phenyl-imidazol-5-carbonsäuren werden durch die allgemeine Formel (I)

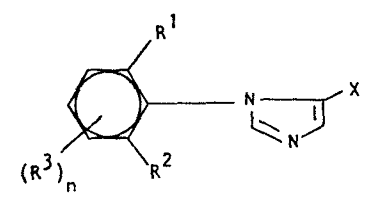

(I)

beschrieben, worin die Symbole folgende Bedeutung haben:
$R^1$, $R^2$ unabhängig voneinander $(C_1\text{-}C_4)$Alkyl;
$R^3$ $(C_1\text{-}C_4)$Alkyl, $(C_1\text{-}C_4)$Alkoxy oder Halogen, wobei bei $n > 1$ die Substituenten auch unterschiedlich sein können;
n 0, 1, 2 oder 3; und
X eine Cyano-, Ester-, Thiolester-, Amid-, Thioester-, Amidoxim-, Alkoxymethyl-, Hydroxymethyl-, Aldehyd-, Keto-, Oxim-, Acetal-, Ketal-, Thioacetal- oder Thioketalgruppe oder ein heterocyclischer Substituent (z.B. Tetrazolyl).

Die neuen Verbindungen weisen eine das Pflanzenwachstum regulierende Wirkung auf. In einem Verfahren zur Herstellung dieser Verbindungen wird von der freien Carbonsäure (X= $-COOH$), ihrem Amid (X= $-CONH_2$), ihren Niederalkylestern [X= $-CO\text{-}O\text{-}(C_1\text{-}C_4)$Aklyl] oder aktivierten Derivaten wie Säurehalogeniden ausgegangen und diese dann derivatisiert. Zu den Verbindungen zählen auch die Salze und Quaternisierungsprodukte des basischen Imidazolrings.

EP 0 185 961 A2

HOECHST AKTIENGESELLSCHAFT HOE 84/F 290 Dr.AU/mk

1-Phenyl-imidazol-5-carbonsäure-Derivate, ein Verfahren zu ihrer Herstellung und ihre Verwendung als Wachstumsregulatoren

Die Erfindung betrifft 1-Phenyl-imidazol-5-carbonsäure-Derivate wie Nitrile (Cyanoverbindungen), Amide oder Thiolester, ein Verfahren zur Herstellung dieser Derivate und deren Verwendung als Wachstumsregulatoren.

Im Phenylring substituierte 1-Phenyl-imidazol-5-carbonsäuren, -carbonsäureamide, -carbonsäureester bzw. -carbonsäurethiolester oder Salze dieser Carbonsäuren und deren Einsatz als Wachstumsregulatoren sind aus der DE-A 32 17 094 bekannt. Diese Verbindungen werden durch die folgende allgemeine Formel beschrieben,

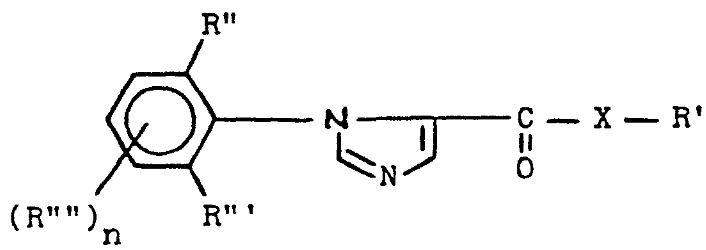

worin die Symbole folgende Bedeutung haben:

X O, S oder N-R';

R' H, Phenyl, $(C_2-C_6)$Alkenyl, unsubstituiertes oder bis zu dreifach durch $(C_1-C_6)$Alkoxy, Di$(C_1-C_3)$alkylamino oder Halogen substituiertes $(C_1-C_{12})$Alkyl, wobei bei X=N-R' zwei gleiche oder verschiedene Reste R' am N gebunden sind; oder ein Metall- oder Ammoniumkation bei X-R' mit $X=O^{\ominus}$ oder $S^{\ominus}$;

R", R"' unabhängig voneinander $(C_1-C_4)$Alkyl;

R"" $(C_1-C_4)$Alkyl, $(C_1-C_4)$Alkoxy oder Halogen, wobei für n > 1 die Substituenten auch unterschiedlich sein können; und

n 0, 1, 2 oder 3.

Einige der bekannten Verbindungen weisen zwar bereits eine ausreichende Wirkung als Wachstumsregulatoren auf, bei ihrem Einsatz müssen jedoch häufig noch verhältnismäßig große Mengen verwendet werden, so daß gelegentlich auch unerwünschte Nebenwirkungen auftreten können.

Aufgabe der vorliegenden Erfindung ist es deshalb, neue wirksame Derivate von 1-Phenyl-imidazol-5-carbonsäuren zu synthetisieren, die auch in geringerer Einsatzmenge als Wachstumsregulatoren angewendet werden können.

Gegenstand der vorliegenden Erfindung sind daher die Verbindungen der Formel (I)

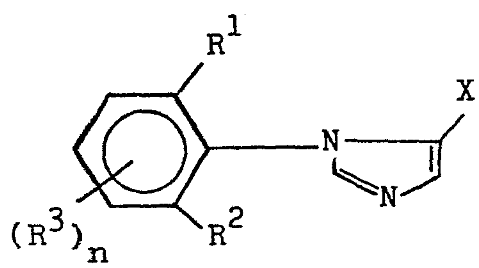

(I),

worin

$R^1$, $R^2$ unabhängig voneinander $(C_1-C_4)$Alkyl;

$R^3$ $(C_1-C_4)$Alkyl, $(C_1-C_4)$Alkoxy oder Halogen, wobei bei n > 1 die Substituenten auch unterschiedlich sein können;

n 0, 1, 2 oder 3;

X -CN, -COOR⁴, -COSR⁵, 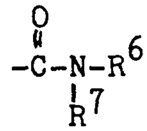, 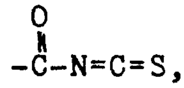,

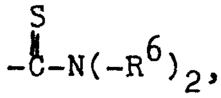, 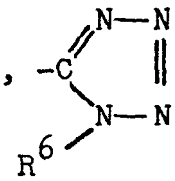, 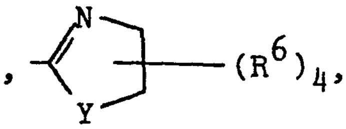,

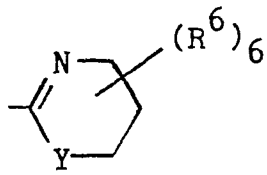 , 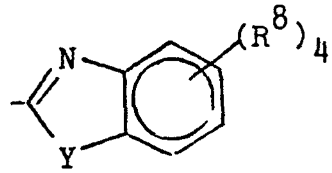 ,

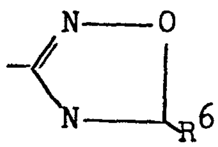 , 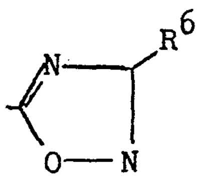 , 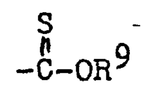 , 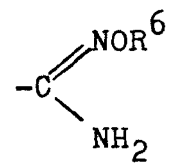

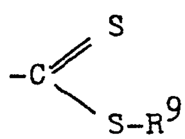 , $-CH_2-OR^{16}$ , 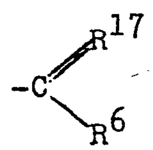 oder

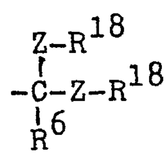 ;

Y O, S oder $N-R^6$;

Z O oder S;

$R^4$ substituiertes $(C_1-C_{12})$Alkyl, vorzugsweise $(C_1-C_6)$Alkyl, das bis zu dreifach, vorzugsweise einfach substituiert ist durch Hydroxy, $(C_1-C_4)$Alkoxy-$(C_1-C_4)$alkoxy, Cyclo$(C_3-C_7)$alkyl, Benzyloxy, Benzyloxyethoxy, Phenyl, Halogenphenyl, Methylphenyl, Cyano, Carbamoyl, Oxiranyl, Mono($C_1$-$C_6$)alkylamino, ($C_1$-$C_4$)Alkylthio, Trimethylsilyl, ($C_1$-$C_4$)Alkanoyl, ($C_1$-$C_4$)Alkoxycarbonyl, Phenoxy, Phenylthio, $-O-N=C(-CH_3)_2$, Triazolyl, Imidazolyl, Carboxyl oder Carboxylat mit einem Ammoniumoder Metallkation, vorzugsweise aus der Gruppe der Alkali- oder Erdalkalimetallkationen, oder mit einem organischen Ammonium-, Phosphonium-, Sulfonium- oder Sulfoxoniumkation;
Halogen($C_3$-$C_6$)alkenyl; unsubstituiertes oder durch Halogen oder ($C_1$-$C_4$)Alkyl substituiertes Cyclo($C_3$-$C_6$)alkyl; unsubstituiertes oder durch Halogen oder Methyl substituiertes Cyclo($C_5$-$C_6$)-alkenyl;($C_3$-$C_6$)Alkinyl; 1,2-Epoxy-prop-3-yl; substituiertes Phenyl, welches ein- oder zweifach substituiert ist durch Halogen, Nitro, Cyano, ($C_1$-$C_4$)Alkyl, ($C_1$-$C_4$)Alkoxycarbonyl oder ($C_1$-$C_4$)Alkoxy; $-N=CR^{10}R^{11}$, $-NR^6R^{12}$

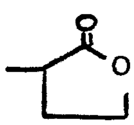 , 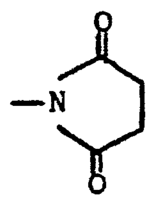 , 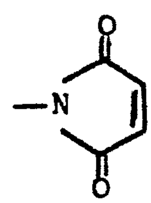 , 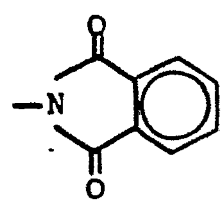 ,

(($C_1$-$C_4$)-Alkyl)carbonyl, Phenylcarbonyl, das durch Halogen , Nitro, Cyano oder ($C_1$-$C_4$)-Alkyl substituiert sein kann, oder einen Rest der Formel

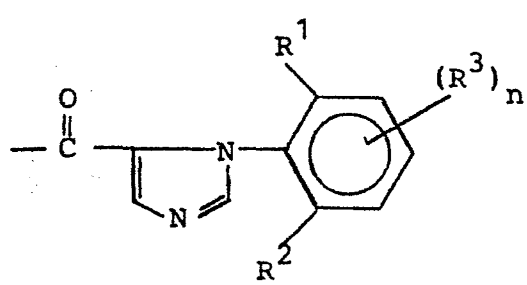

$R^5$ substituiertes $(C_1-C_{12})$Alkyl, vorzugsweise $(C_1-C_6)$Alkyl, das bis zu zweifach, vorzugsweise einfach substituiert ist durch $(C_1-C_4)$Alkoxyethoxy, Cyclo$(C_3-C_6)$alkyl, Benzyloxy, Phenyl, $(C_1-C_4)$Alkylthio, $(C_1-C_4)$Alkoxycarbonyl, Phenoxy, Carboxyl oder Carboxylat mit einem Ammonium- oder Metallkation, vorzugsweise aus der Gruppe der Alkali- oder Erdalkalimetallkationen, oder mit einem organischen Ammonium-, Phosphonium-, Sulfonium- oder Sulfoxoniumkation; oder Cyclo$(C_3-C_6)$alkyl;

$R^6$ H oder $(C_1-C_4)$Alkyl;

$R^7$ substituiertes $(C_1-C_{12})$Alkyl, vorzugsweise $(C_1-C_6)$Alkyl, das bis zu zweifach, vorzugsweise einfach substituiert ist durch $(C_1-C_4)$Alkoxyethoxy, Hydroxyl, Cyclo$(C_3-C_6)$alkyl, Benzyloxy, Phenyl, Cyano, Carbamoyl, Carboxyl, $(C_1-C_4)$Alkoxycarbonyl, Formyl oder Phenoxy; Cyclo$(C_3-C_6)$alkyl; bis zu zweifach durch Halogen, Nitro, Cyano, $(C_1-C_4)$Alkyl oder $(C_1-C_4)$-Alkoxy subsituiertes Phenyl;
$-NR^6R^{13}$, $-OR^{14}$, $-NH-CO-NH_2$, $-NH-CS-NH_2$ oder $-SO_2R^{15}$;

oder $R^6$ und $R^7$ bilden - gemeinsam mit dem Stickstoffatom an das sie gebunden sind - einen gesättigten oder ungesättigten, gegebenenfalls benzokondensierten drei- bis siebengliedrigen Ring, der bis zu drei Heteroatome aus der Gruppe O,N und S enthält und der unsubstituiert oder durch $(C_1-C_4)$Alkyl oder Halogen substituiert ist und eine Oxogruppe enthalten kann;

$R^8$ unabhängig voneinander H, Halogen, $(C_1-C_4)$-Alkyl, Nitro oder Cyano;

$R^9$ H, $(C_1-C_6)$Alkyl oder Phenyl, oder bei Thiocarboxylaten ein Alkali- oder Erdalkalimetallkation, Ammonium- oder organisches Ammoniumkation;

$R^{10}$, $R^{11}$ unabhängig voneinander H, unsubstituiertes oder durch Triazolyl oder Imidazolyl substituiertes $(C_1-C_6)$Alkyl; Cyclo$(C_3-C_6)$-alkyl, $(C_3-C_6)$Alkenyl, Phenyl oder Benzyl;

oder sie bilden gemeinsam mit dem Kohlenstoffatom, an das sie gebunden sind, ein unsubstituierte oder durch Methyl oder Halogen substituiertes Cyclo$(C_5-C_7)$alkyl;

$R^{12}$ $(C_1-C_4)$Alkyl, Phenyl, $(C_1-C_6)$Alkylcarbonyl, Benzyl, Benzoyl, Halogenbenzyl, Halogenbenzoyl oder Methylbenzoyl;

$R^{13}$ H, $(C_1-C_4)$Alkyl, Formyl, $(C_1-C_6)$Alkylcarbonyl, Benzoyl, Halogenbenzoyl, Methylbenzoyl oder Trihalogenacetyl;

$R^{14}$ H, $(C_1-C_4)$Alkyl oder Benzyl;

$R^{15}$ $(C_1-C_4)$Alkyl, Phenyl, Chlorphenyl oder Methylphenyl,

$R^{16}$ H, unsubstituiertes oder durch Phenyl oder $(C_1-C_4)$-Alkylphenyl substituiertes $(C_1-C_{12})$-alkyl; $(C_1-C_6)$Alkylcarbonyl, Halogen$(C_1-C_6)$-alkylcarbonyl, N-$(C_1-C_6)$Alkylcarbamoyl, Benzoyl, Halogenbenzoyl oder Methylbenzoyl;

$R^{17}$ O (einschließlich der Bisulfidaddukte) oder $=N-O-R^{16}$; und

$R^{18}$ unabhängig voneinander unsubstituiertes oder durch Phenyl, Cyclo($C_5$-$C_7$)alkyl, ($C_1$-$C_4$)Alkoxy, ($C_1$-$C_4$)Alkylthio oder Halogen substituiertes ($C_1$-$C_6$)Alkyl; oder beide Reste $R^{18}$ bilden, gemeinsam mit Z und dem Kohlenstoffatom an das sie gebunden sind, einen unsubstituierten oder durch ($C_1$-$C_4$)Alkyl, Hydroxy-($C_1$-$C_4$)alkyl, Halogen-($C_1$-$C_4$)alkyl oder Phenyl substituierten 5- oder 6-gliedrigen Ring

bedeuten sowie deren für die Landwirtschaft einsetzbaren Salze oder Quaternisierungsprodukte.

Dabei können die bei der Definition der allgemeinen Formel (I) vorkommenden Alkyl-, Alkenyl- und Alkinylreste sowohl geradkettig als auch verzweigt sein; unter Halogen ist F, Cl, Br oder J, insbesondere F, Cl oder Br zu verstehen.

Die Salzbildung oder Quaternisierung erfolgt am basischen Imidazolring der Verbindungen der Formel I. Diese Derivatisierung ist jedoch, im Falle $R^4$, $R^5$ oder $R^9$ = ein Kation bedeutet, nicht möglich. Für den Fall, daß in den aufgeführten Substituenten - zusätzlich zum Imidazolring - weitere basische Stickstoffatome auftreten, ist auch eine mehrfache Salzbildung oder Quaternisierung möglich.

Bevorzugt unter den Verbindungen der Formel I sind solche, bei denen

$R^1$, $R^2$ unabhängig voneinander ($C_1$-$C_3$)Alkyl,

$R^3$ Methyl oder Halogen,

n 0 oder 1,

X $-COOR^4$, 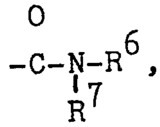, 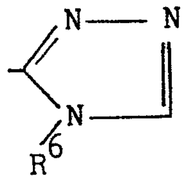,

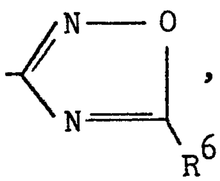, 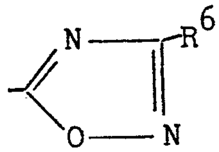, 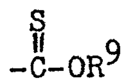, 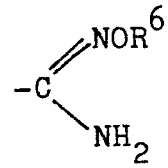

oder 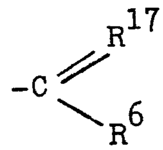

$R^4$ $(C_1-C_6)$Alkyl, das einfach durch Hydroxy substituiert ist, $(C_1-C_4)$Alkylthio, Cyclo$(C_3-C_6)$alkyl, $C_3$-Alkinyl, einen Rest der Formeln $-N{=}CR^{10}R^{11}$ oder

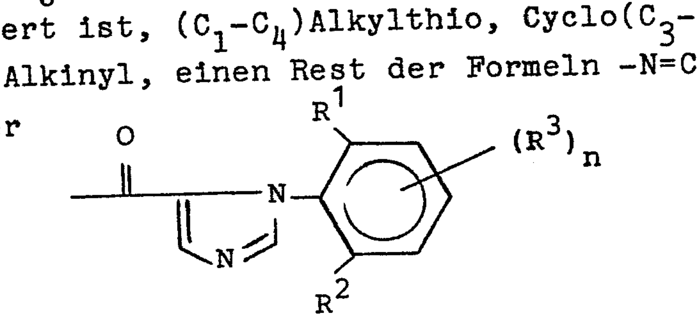

$R^6$ H oder Methyl,

$R^7$ $(C_1-C_6)$-Alkyl, das einfach durch Hydroxy, Cyano, Carboxy, $(C_1-C_4)$Alkoxycarbonyl substituiert ist, einen Rest der Formeln $-NR^6R^{13}$, $-OR^{14}$ oder $-SO_2R^{15}$; oder

$R^6$ und $R^7$ bilden gemeinsam mit dem Stickstoffatom, an das sie gebunden sind, einen heterocyclischen Ring der Formeln

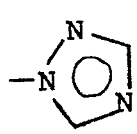 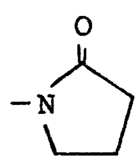 oder 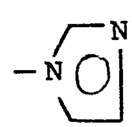,

$R^9$ $(C_1-C_6)$-Alkyl,

$R^{10}$, $R^{11}$ Methyl,

$R^{13}$, $R^{14}$ H,

$R^{15}$ Chlorphenyl und

$R^{17}$ O (einschließlich der Bisulfidaddukte)

bedeuten, sowie deren Salze und Quaternisierungsprodukte .

Gegenstand der Erfindung sind außerdem Verfahren zur Herstellung der Verbindungen der allgemeinen Formel (I):

Die Cyanoverbindung (X =-CN) erhält man, indem man das an sich bekannte Carbonsäureamid (X =-$CONH_2$, siehe DE-A 32 17 094) dehydratisiert. Als Mittel hierzu kommen beispielsweise Phosphorpentachlorid, Phosphoroxychlorid, Thionylchlorid, Phosphorpentoxid und Acetanhydrid infrage. Die Reaktionstemperatur hängt stark von der Wahl des Dehydratisierungsmittels ab und liegt im allgemeinen zwischen 20 und 120°C. Gegebenenfalls können auch inerte organische Lösemittel zugesetzt werden.

Die Ester, Thiolester oder Amidderivate (X=-$COOR^4$, -$COSR^5$, -$CONR^6R^7$) lassen sich aus den Carbonsäuren (siehe DE-A 32 17 094) oder den daraus erhältlichen aktivierten Verbindungen wie Säurehalogeniden oder gemischte Kohlensäureanhydriden durch Umsetzung mit den entsprechenden Alkoholen, Thiolen oder Aminverbindungen herstellen. Ester und Thiolester lassen sich daneben auch durch Alkylierung der Carbonsäuren mit Halogenalkylverbindungen in Gegenwart von Basen erhalten.

Die Tetrazole sind durch Umsetzung der Nitrile (X=-CN) mit Aziden in organischen Lösemitteln wie DMF oder DMSO bei Temperaturen von 20°C bis 150°C erhältlich. Die anderen heterocyclischen Verbindungen der Formel (I) mit X =

Die Tetrazole sind durch Umsetzung der Nitrile (X=-CN) mit Aziden in organischen Lösemitteln wie DMF oder DMSO bei Temperaturen von 20°C bis 150°C erhältlich. Die anderen heterocyclischen Verbindungen der Formel (I) mit X =

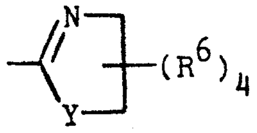 , 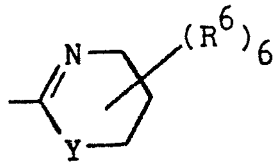 , 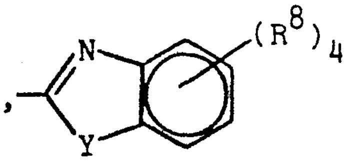 ,

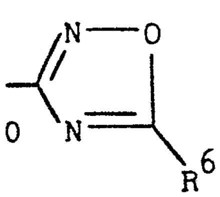 und 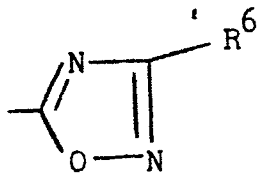 sind durch Umsetzung der entsprechenden Carbonsäuren (siehe DE-A 32 17 094) beziehungsweise deren aktivierten Vertretern wie ihren Säurechloriden mit bifunktionellen Verbindungen wie Diaminen, Aminoalkoholen, Aminothiolen oder Amidoximen nach an sich bekannten Verfahren zugänglich (siehe beispielsweise JP-A 57/175 177 und 57/175 179).

Die Thioester (X=-CSOR$^9$, -CSSR$^9$) lassen sich aus den entsprechenden Sauerstoffverbindungen (Ester) durch Umsetzung mit Phosphorpentasulfid, vorteilhaft in Gegenwart von Basen, in organischen Lösemitteln wie Dimethylformamid, Acetonitril, Toluol oder Xylol erhalten.

Die Amidoxime 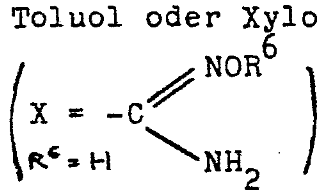 werden aus den Nitrilen (X = -CN) durch Umsetzung mit Hydroxylamin erhalten und können durch Umsetzung mit Alkylierungsmitteln in die anderen Derivate mit R$^6$≠H umgewandelt werden.

Die Alkohole (X = -CH$_2$-O-R$^{16}$ mit R$^{16}$ = H) lassen sich durch Reduktion aus den bekannten Säuren und Estern (siehe DE-A 32 17 094) herstellen. Als vorteilhaft erwies sich zum Beispiel die Reduktion der Niederalkylester mit komplexen

Hydriden wie Lithiumaluminiumhydrid in Tetrahydrofuran oder Diethylether. Die Hydroxylgruppe läßt sich dann nach üblichen Verfahren wie Alkylierung, Acylierung oder Carbamoylierung in die anderen Verbindungen mit X = $-CH_2-OR^{16}$ und $R^{16}$ ≠ H überführen.

Die Aldehydderivate ( 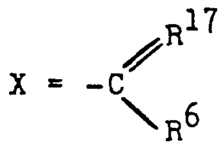 mit $R^6$ = H, $R^{17}$=O) sind durch selektive Oxidation der Alkohole ($X=-CH_2-OR^{16}$ mit $R^{16}$ = H) zugänglich. Als Oxidationsmittel sind beispielsweise Dimethylsulfoxid in Gegenwart von Oxalylchlorid oder Acetanhydrid geeignet. Die Ketonderivate ( 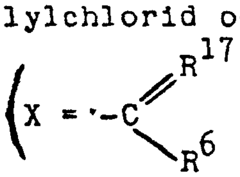 mit $R^6$ = $(C_1-C_4)$Alkyl, $R^{17}$ = O) werden durch Umsetzung geeigneter Dialkylamide der Carbonsäuren mit Grignardreagentien erhalten (siehe Synthesis (1984), S. 228 f). Die weiteren Derivatisierungen zu den Oximderivaten ( 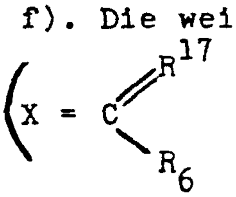 mit $R^{17}$ = $=NOR^{16}$) oder den Acetal-, Ketal-, Thioacetal- oder Thioketalverbindungen ( 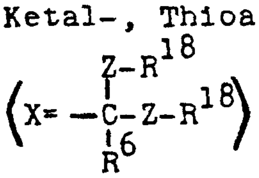 ) sind nach gängigen Methoden wie Umsetzung mit Hydroxylaminhydrochlorid in Gegenwart von anorganischen oder organischen Basen und gegebenenfalls anschließender Alkylierung, Acylierung oder Carbamoylierung, oder Umsetzung mit Alkoholen oder Thiolen unter Wasserabspaltung möglich.

Erfindungsgemäß verwendbare Verbindungen sind – wie bereits vorstehend definiert – auch die Salze der Verbindungen der Formel (I) und die Quaternisierungsprodukte der Verbindungen der Formel (I).

Zur Herstellung der Salze der Verbindungen der Formel (I) kommen folgende Säuren in Frage: Halogenwasserstoffsäuren wie Chlorwasserstoffsäure oder Bromwasserstoffsäure, ferner Phosphorsäure, Salpetersäure, Schwefelsäure, mono- und bifunktionelle Carbonsäuren und Hydroxycarbonsäuren wie Essigsäure, Maleinsäure, Bernsteinsäure, Fumarsäure, Weinsäure, Citronensäure, Salicylsäure, Sorbinsäure oder Milchsäure, sowie Sulfonsäuren wie p-Toluolsulfonsäure oder 1,5-Naphthalindisulfonsäure. Die Säureadditionssalze der Verbindungen der Formel (I) können in einfacher Weise nach üblichen Salzbildungsmethoden, z.B. durch Lösen einer Verbindung der Formel (I) in einem geeigneten organischen Lösemittel und Hinzufügen der Säure erhalten werden und in bekannter Weise, z.B. durch Abfiltrieren, isoliert und gegebenenfalls durch Waschen mit einem inerten organischen Lösemittel gereinigt werden.

Zur Herstellung von Quaternisierungsprodukten verwendet man Alkyl-, Alkylthioalkyl-, Alkoxyalkyl-, insbesondere $(C_1-C_6)$Alkyl- und gegebenenfalls im Phenylrest substituierte, insbesondere halogenierte Phenacylhalogenide. Die Herstellung dieser Derivate erfolgt nach den allgemein üblichen Methoden.

Mit den erfindungsgemäßen Verbindungen sind typische wachstumsregulierende Effekte erzielbar, die - verglichen mit den aus der DE-A 32 17 094 bekannten Verbindungen - in einigen Fällen bereits bei niedrigeren Dosierungen einsetzen können. Sie greifen regulierend in den pflanzeneigenen Stoffwechsel ein und können damit zur gezielten Beeinflussung von Pflanzeninhaltsstoffen sowie zur Ernteerleichterung wie zum Auslösen von Desikkation und Wuchsstauchung eingesetzt werden. Des weiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann. Besonders hervorzuheben ist die wachstumsregulatorische Wirksamkeit der Verbindungen als Wuchshemmer in Getreide, Mais, Soja, Tabak, Baumwolle, Ackerbohne, Raps, Reis, Rasen sowie ihre Fähigkeiten, den Gehalt an erwünschten Inhaltsstoffen wie Kohlehydraten (z.B. in Zuckerrohroder Hirsekulturen) und Protein bei Nutzpflanzen zu erhöhen. Schließlich zeigen die Verbindungen eine sehr gute Verbesserung der Fruchtabszission, insbesondere bei Zitrusfrüchten.

Eine weitere Lösung der gestellten Aufgabe sind auch das Pflanzenwachstum regulierende Mittel, die sich durch einen wirksamen Gehalt mindestens einer der erfindungsgemäßen Verbindungen auszeichnen. Die Aufwandmenge der erfindungsgemäßen Verbindungen beträgt im allgemeinen 0,02 bis 2,5 kg Wirksubstanz pro ha, vorzugsweise 0,05 bis 1,5 kg/ha. Die erfindungsgemäßen Verbindungen lassen sich bei ihrem praktischen Einsatz gegebenenfalls auch vorteilhaft mit bekannten Wachstumsregulatoren oder natürlichen oder pflanzlichen Hormonen kombinieren.

Die erfindungsgemäßen Verbindungen können, gegebenenfalls im Gemisch mit weiteren Wirkkomponenten, als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem (den) Wirkstoff(en) außer gegebenenfalls einem Verdünnungs- oder Inertstoff noch Netzmittel wie polyoxethylierte Fettalkohole, Alkyl- oder Alkylphenylsulfonate und/oder Dispergierhilfsmittel wie ligninsulfonsaures Natrium, 2,2'-dinaphthylmethan-6,6'-disulfonsaures Natrium, dibutylnapthalinsulfonsaures Natrium oder auch oleoylmethyltaurinsaures Natrium enthalten. Die Herstellung erfolgt in üblicher Weise, z.B. durch Mahlen und Vermischen der Komponenten.

Emulgierbare Konzentrate können z.B. durch Auflösen des (der) Wirkstoffe(s) in einem inerten organischen Lösemittel wie Butanol, Cyclohexanon, Dimethylformamid, Xylol oder auch höhersiedenden Aromaten oder aliphatischen oder cycloaliphatischen Kohlenwasserstoffen unter Zusatz von einem oder mehreren Emulgatoren hergestellt werden. Bei flüssigen Wirkstoffen kann der Lösemittelanteil auch ganz oder teilweise entfallen. Als Emulgatoren können beispielsweise verwendet werden: Alkyl-arylsulfonsaure Calciumsalze wie Cadodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des (der) Wirkstoffe(s) mit feinverteilten, festen Stoffen, z.B. Talkum, natürlichen Tonen wie Kaolin, Bentonit, Pyrophillit oder Diatomeenerde erhalten. Granulate können entweder durch Verdüsen des (der) Wirkstoffe(s) auf adsorptionsfähiges, granuliertes Inertmaterial hergestellt werden oder durch Aufbringen von Wirkstoffkonzentrationen mittels Bindemitteln, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 10 bis 80 Gew.-% betragen. Staubförmige Formulierungen enthalten meistens 5 bis 20 Gew.-% an Wirkstoff(en), versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden. Daneben enthalten die genannten Wirkstofformulierungen gegebenenfalls die jeweils üblichen Haft-, Netz-, Dispergier-, Emulgier-, Penetrations-, Lösemittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen mit anderen Wirkstoffen wie Insektiziden, Akariziden, Herbiziden, Düngemitteln oder Fungiziden sind gegebenenfalls möglich.

In den nachfolgenden Beispielen verhalten sich Gew.-Teile (GT) zu Vol.-Teilen (VT) wie kg zu $dm^3$ (l) und %-Angaben beziehen sich - falls nichts anderes angegeben ist - auf das Gewicht.

## Formulierungsbeispiele

### Beispiel 1

Ein Stäubemittel wird erhalten, indem man a) 10 GT Wirkstoff(e) und 90 GT Talkum oder einem anderen Inertstoff mischt und in einer Schlagmühle zerkleinert, oder indem man b) 60 GT Wirkstoff, 35 GT Talkum und 5 GT Haftmittel (z.B. ein Polysaccharid wie (R)Rhodopol der Rhone-Poulenc S.A.) in der gleichen Weise homogenisiert.

### Beispiel 2

Ein in Wasser leicht dispergierbares, benetzbares Pulver wird erhalten, indem man 25 GT Wirkstoff(e), 64 GT kaolinhaltigen Quarz als Inertstoff, 10 GT ligninsulfonsaures Kalium und 1 GT oleoylmethyltaurinsaures Natrium als Netz- und Dispergierhilfsmittel mischt und in einer Stiftmühle mahlt. Eine Formulierung mit 5 % Wirkstoffgehalt kann wie folgt zusammengesetzt sein: 5 % Wirkstoff(e), 6 % eines sulfonierten Napthalinformaldehydkondensats (z.B. (R)Dispersogen A der HOECHST AG), 2 % eines Na-Salzes einer Alkylnapthalinsulfonsäure (z.B. (R)Leonil DB der HOECHST AG), 5 % eines Gemisches aus Polypropylenglykol und $SiO_2$ (z.B. (R)Acrotin 341 der HOECHST AG), 25 % eines $SiO_2$ (z.B. (R)Sipernat der Degussa AG) und 57 % Kaolin Typ 1777.

### Beispiel 3

Ein in Wasser leicht dispergierbares Dispersionskonzentrat wird erhalten indem man 20 GT Wirkstoff(e) mit 6 GT eines Alkylphenolpolyglykolethers (z.B. (R)Triton X 207 von Rohm and Haas Co.), 3 GT Isotridecanolpolyglykolether (8 Ethylenoxid-Einheiten) und 71 GT paraffinischem Mineralöl (Siedebereich ca. 255 bis über 377°C) mischt und in einer Reibkugelmühle auf eine Feinheit von unter 5 µm vermahlt.

Beispiel 4

Ein emulgierbares Konzentrat wird erhalten aus 15 GT Wirkstoff(e), 75 GT Cyclohexanon als Lösemittel und 10 GT oxethyliertem Nonylphenol (10 Ethylenoxid-Einheiten) als Emulgator.

Chemische Beispiele

Beispiel 1

5-Cyano-1-(2,6-diethylphenyl)-imidazol

36,3 GT (0,15 mol) 1-(2,6-Diethylphenyl)-imidazol-5-carboxamid werden bei Raumtemperatur portionsweise zu 110 VT Phosphoroxychlorid gegeben. Nach 2 Stunden bei 80°C wird im Vakuum eingedampft, der Rückstand auf Eis gegeben, mit Natronlauge neutralisiert, und zweimal mit Toluol extrahiert. Nach dem Trocknen über Natriumsulfat wird die organische Phase eingedampft und im Vakuum destilliert. Man erhält 27,2 GT (81 % d. Theorie) an 5-Cyano-1-(2,6-diethylphenyl)-imidazol vom Siedepunkt (Kp) 133-5°C/0,01 mbar.

Beispiel 2

Pthalimido-1-(2,6-diethylphenyl)-imidazol-5-carboxylat

Zu 6,0 GT (0,020 mol) 1-(2,6-Diethylphenyl)-imidazol-5-carbonylchlorid-Hydrochlorid (hergestellt aus 1-(2,6-Diethylphenyl)-imidazol-5-carbonsäure und Thionylchlorid bei 80°C) und 3,7 GT (0,0023 mol) N-Hydroxy-phthalimid in 50 VT absolutem Acetonitril gibt man 4,1 GT (0,029 mol) $K_2CO_3$ portionsweise zu. Man erhitzt 1 h auf 60°C, läßt abkühlen, gießt auf Wasser und saugt ab. Man erhält 6,9 GT (88 % d. Theorie) an Phthalimido-1-(2,6-diethylphenyl)-imidazol-5-carboxylat, einem farblosen Feststoff vom Schmelzpunkt (Fp) 179-80°C.

Beispiel 3

N-(2-Hydroxyethyl)-1-(2,6-diethylphenyl)-imidazol-5-carboxyamid

Zu 25,0 GT (0,083 mol) 1-(2,6-Diethylphenyl)-imidazol-5-carbonylchlorid-Hydrochlorid (siehe Beispiel 2) in 50 VT Acetonitril tropft man bei Raumtemperatur ein Gemisch aus 5,5 GT (0,091 mol) Aminoethanol und 18,4 GT (0,18 mol) Triethylamin zu. Nach 1 h wird auf Wasser/Toluol gegeben, die organische Phase über Natriumsulfat getrocknet und eingedampft. Man erhält 16,1 GT (67,5 % d. Theorie) an N-(2-Hydroxyethyl)-1-(2,6-diethylphenyl)-imidazol-5-carboxamid, einem Feststoff vom Fp. 165-8°C.

Beispiel 4

Methylthiomethyl-1-(2,6-diethylphenyl)-imidazol-5-carboxylat

20,0 GT (0,082 mol) 1-(2,6-Diethylphenyl)-imidazol-5-carbonsäure, 7,9 GT (0,082 mol) Chlordimethylsulfid und 14,7 GT (0,11 mol) Kaliumcarbonat werden in 100 VT absolutem Acetonitril 1 h unter Rückfluß erhitzt. Man läßt erkalten, gießt in 200 VT Wasser und extrahiert das Produkt zweimal mit je 100 VT Toluol. Nach dem Trocknen und Eindampfen erhält man 20,1 GT (80,1 % d. Theorie) an Methylthiomethyl-1-(2,6-diethylphenyl)-imidazol-5-carboxylat, einem hellgelben Öl. Die Identifizierung erfolgt $^{1}$H-NMR-spektroskopisch.

Beispiel 5

1-(2,6-Diethylphenyl)-5-(5-tetrazolyl)-imidazol

10,0 GT (0,044 mol) 5-Cyano-1-(2,6-diethylphenyl)-imidazol (siehe Beispiel 1), 2,4 GT (0,44 mol) Ammoniumchlorid

und 2,9 GT (0,044 mol) Natriumazid werden 24 h bei 100°C in 50 VT absolutem Dimethylformamid gerührt. Nach dem Erkalten wird auf 200 VT Wasser gegeben, mit Eisessig auf pH 5 angesäuert und der Niederschlag abgesaugt. Man erhält 5,7 GT (48 % d. Theorie) an 1-(2,6-Diethylphenyl)-5-(tetrazolyl)-imidazol, einem hellgelben Feststoff vom Fp 109-13°C.

Beispiel 6

1-(2,6-Diethylphenyl)-5-(4,4-dimethyl-2-oxazolin-2-yl)-imidazol

Zu 19,5 GT (0,062 mol) N-2-(1-Hydroxy-2-methyl-propyl)-1-(2,6-diethylphenyl)-imidazol-5-carboxamid in 60 VT absolutem Methylenchlorid tropft man bei Raumtemperatur 44 GT (0,37 mol) Thionylchlorid zu. Nach 1 h wird im Vakuum eingedampft, der Rückstand in 100 GT Wasser aufgenommen, das Gemisch mit Natriumbicarbonat neutralisiert, mit Toluol extrahiert und nach dem Trocknen ($Na_2SO_4$) und Eindampfen über eine Kieselgelsäule chromatografiert (Laufmittel: Petrolether/Essigsäure 7:3). Man erhält 13,3 GT (72 % d. Theorie) an 1-(2,6-Diethylphenyl)-5-(4,4-dimethyl-2-oxazolin-2-yl)-imidazol, einem farblosen Öl. Die Identifizierung erfolgt $^1$H-NMR-spektroskopisch.

Beispiel 7

1-(2,6-Diethylphenyl)-5-(3-methyl-1,2,4-oxdiazol-5-yl)-imidazol

Zu 5,1 GT (0,047 mol) Acetamidoxim-Hydrochlorid in 50 VT Pyridin gibt man bei 0 bis 10°C 10,0 GT (0,033 mol) 1-(2,6-Diethylphenyl)-imidazol-5-carbonylchlorid-Hydrochlorid portionsweise zu. Nach zweistündigem Rühren bei

0185961

Raumtemperatur gibt man auf Wasser und saugt ab. Man erhält 6,6 GT (50 % d. Theorie) an 1-(2,6-Diethylphenyl)-5-(3-methyl-1,2,4-oxdiazol-5-yl)-imidazol vom Fp 149-53°C.

Beispiel 8

1-(2,6-Diethylphenyl)-imidazol-5-carboxamidoxim

18,6 GT (0,083 mol) 5-Cyano-1-(2,6-diethylphenyl)-imidazol werden in 50 VT Ethanol gelöst, bei Raumtemperatur wird eine Lösung von 6,9 GT (0,99 mol) Hydroxylammoniumchlorid und 6,9 GT (0,05 ml) Kaliumcarbonat in 10 VT Wasser zugetropft. Man rührt 2 h bei 80°C, läßt abkühlen, nimmt in 300 VT Toluol auf, wäscht zweimal mit Wasser, trocknet und dampft ein. Man erhält 19,7 GT (92 % d. Theorie) an 1-(2,6-Diethylphenyl)-imidazol-5-carboxamidoxim, einem leicht hellgelben Feststoff vom Fp 107-12°C.

Beispiel 9

1-(2,6-Diethylphenyl)-5-(5-methyl-1,2,4-oxdiazol-3-yl)-imidazol

15,0 GT (0,045 mol) O-Chloracetyl-1-(2,6-diethylphenyl)-imidazol-5-carboxamidoxim werden 2 h in 50 VT Eisessig bei 100°C gerührt. Man läßt abkühlen, nimmt in 200 VT Wasser/200 VT Toluol auf, neutralisiert mit Natriumhydrogencarbonat und trennt die organische Phase ab. Nach dem Trocknen, Eindampfen und Chromatografieren (Kieselgel, Laufmittel: Petrolether/Essigester 1:1) erhält man 7,3 GT (61 % d. Theorie) an 1-(2,6-Diethylphenyl)-5-(5-methyl-1,2,4-oxdiazol-3-yl)-imidazol, einem farblosen Öl. Die Identifizierung erfolgt $^{1}$H-NMR-spektroskopisch.

Beispiel 10

N-Methylsulfonyl-1-(2,6-diethylphenyl)-imidazol-5-carboxamid

Zu einem Gemisch aus 13,6 GT (0,14 mol) Methansulfonamid, 24 GT (0,24 mol) Triethylamin und 100 VT absolutem Acetonitril gibt man bei Raumtemperatur 25 GT (0,08 mol) 1-(2,6-Diethylphenyl)-imidazol-5-carbonylchlorid-Hydrochlorid portionsweise zu. Nach 2 h wird auf Wasser gegeben, auf pH 4 angesäuert und abgesaugt. Man erhält 21,1 GT (79 % d. Theorie) an N-Methylsulfonyl-1-(2,6-diethylphenyl)-imidazol-5-carboxamid, einem Feststoff vom Fp 240-2°C.

Beispiel 11

5-(2-Benzimidazolyl)-1-(2,6-diethylphenyl)-imidazol

15,0 GT (0,045 mol) N-(2-Aminophenyl)-1-(2,6-diethylphenyl)-imidazol-5-carboxamid werden mit 0,5 GT p-Toluolsulfonsäure in 100 VT Xylol am Wasserabscheider erhitzt. Man läßt abkühlen, wäscht zweimal mit Wasser, trocknet und dampft ein. Man erhält 13,7 GT (96 % d. Theorie) an 5-(2-Benzimidazolyl)-1-(2,6-diethylphenyl)-imidazol.

Beispiel 12

1-(2,6-Diethylphenyl)-5-hydroxymethyl-imidazol

Zu 7,60 GT (0,20 mol) Lithiumalanat in 150 VT absolutem Tetrahydrofuran tropft man bei Raumtemperatur 54,8 GT (0,20 mol) Ethyl-1-(2,6-diethylphenyl)-imidazol-5-carboxylat zu. Nach 1 h werden vorsichtig 7,6 GT Wasser, 7,6 GT einer 15 %igen wäßrigen NaOH-Lösung und 22,8 GT Wasser zugetropft. Nach dem Absaugen des anorganischen Salzes wird die organische Phase eingedampft und man erhält 45,5 GT (99 % d. Theorie) an 1-(2,6-Diethylphenyl)-5-hydroxymethyl-imidazol, farblosen Nadeln vom Fp 79-82°C.

Beispiel 13

1-(2,6-Diethylphenyl)-5-formyl-imidazol

Zu 500 VT absolutem Methylenchlorid und 32,5 GT (0,26 mol) Oxalylchlorid tropft man bei -78°C 26,4 GT (0,33 mol) absolutes Dimethylsulfoxid in 50 VT absolutem Methylenchlorid zu. Nach 1/2 h bei -78°C wird eine Lösung von 39,0 g (0,17 mol) 1-(2,6-Diethylphenyl)-5-hydroxymethyl-imidazol in 50 VT absolutem Methylenchlorid zugetropft. Nach 2 h bei -70°C werden 85 GT (0,85 mol) Triethylamin zugetropft. Nach dem Erwärmen auf Raumtemperatur wäscht man die organische Phase zweimal mit Wasser, trocknet ($Na_2SO_4$) und dampft ein. Man erhält 38,5 GT (98 % d. Theorie) an 1-(2,6-Diethylphenyl)-5-formyl-imidazol, einem hellgelben Öl. Die Identifizierung erfolgt $^1$H-NMR-spektroskopisch.

Beispiel 14

1-(2,6-Diethylphenyl)-5-hydroximinomethyl-imidazol

Zu 16,8 GT (0,074 mol) 1-(2,6-Diethylphenyl)-5-formyl-imidazol in 30 VT Pyridin gibt man bei Raumtemperatur 7,7 GT (0,11 mol) Hydroxylammoniumchlorid portionsweise zu. Nach 4 h wird auf 500 VT Wasser gegossen und der Feststoff abgesaugt. Man erhält 13,0 GT (73 % d. Theorie) an 1-(2,6-Diethylphenyl)-5-hydroximinomethyl-imidazol vom Fp 180-3°C.

Beispiel 15

5-Acetyl-1-(2,6-diethylphenyl)-imidazol

Zu einer aus 5,9 GT (0,042 mol) Methyljodid und 1,0 GT (0,042 mol) Magnesium in 100 VT absolutem Tetrahydrofuran hergestellten Methylmagnesiumbromidlösung tropft man bei Raumtemperatur eine Lösung von 8,7 GT (0,032 mol) N,N-Dimethyl-1-(2,6-diethylphenyl)-imidazol-5-carboxamid in 50 VT absolutem Tetrahydrofuran zu. Nach 1,5 h wird auf Ammoniumchloridlösung gegossen und das Produkt zweimal mit Ether extrahiert. Nach dem Trocknen ($Na_2SO_4$) wird eingedampft. Man erhält 7,1 GT (91 % d. Theorie) an 5-Acetyl-1-(2,6-diethylphenyl)-imidazol, einem farblosen Öl. Die Identifizierung erfolgt $^1H$-NMR-spektroskopisch.

Weitere Beispiele sind in Tabelle 1 aufgeführt.

Tabelle 1    Imidazolderivate der Formel (I)

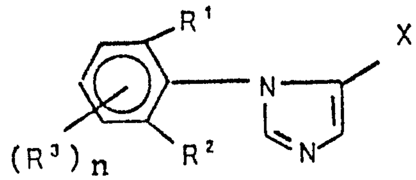

(I)    n = O oder 1

| Bsp. Nr. | $R^1$ | $R^2$ | $R^3$ | -X | Fp. [°C] | hergestellt nach Beispiel |
|---|---|---|---|---|---|---|
| 16 | $CH_3$ | $CH_3$ | – | -COO-Cyclohexyl | | 2 |
| 17 | " | " | – | $-COO-CH_2-CCl=CH_2$ | | 4 |
| 18 | " | " | $4-CH_3$ 1) | -COO-(4-Cl-Phenyl) | | 2 |
| 19 | " | " | " 1) | $-C(NH_2)=NOCH_3$ | | 8 |
| 20 | " | " | " 1) | $-COO-CH_2-CH_2$-Cyclohexyl | | 2 |
| 21 | " | $C_2H_5$ | – | $-C(=S)OC_2H_5$ | | – 2) |
| 22 | " | " | – | $-C(=S)SC_2H_5$ | | – 2) |
| 23 | " | " | $3-CH_3$ 1) | $-C(=S)N(CH_3)_2$ | | 2 |
| 24 | " | " | " 1) | -CONH-NH-CO-Phenyl | | 3 |
| 25 | " | " | " 1) | $-CONH-NH-COCH_3$ | | 3 |
| 26 | $C_2H_5$ | " | 4-Br 1) | $-CONH-OCH_3$ | | 3 |
| 27 | " | " | " 1) | $-COO-NH-COCH_3$ | | 2 |
| 28 | " | " | – | $-COO(-CH_2)_2-O(-CH_2)_2-O-CH_3$ | Oel | 2 |
| 29 | " | " | – | $-COO-CH_2$-Phenyl | " | 2 |

Fortsetzung Tabelle 1

| Bsp. Nr. | $R^1$ | $R^2$ | $R^3$ | -X | Fp. [°C] | hergestellt nach Beispiel |
|---|---|---|---|---|---|---|
| 30 | $C_2H_5$ | $C_2H_5$ | – | $-COO-CH(CH_3)CN$ | | 2 |
| 31 | " | " | – | $-COOCH_2-CONH_2$ | | 4 |
| 32 | " | " | – | $-COO-CH_2-CO-CH(CH_3)_2$ | | 4 |
| 33 | " | " | – | $-COO-CH_2-COOCH_3$ | 60-3 | 4 |
| 34 | " | " | – | $-COO-CH(CH_3)-COOCH_3$ | Oel | 4 |
| 35 | " | " | – | $-COO-CH_2$-Triazol | | 4 |
| 36 | " | " | – | -COO-Cyclohexyl | Oel | 2 |
| 37 | " | " | – | $-COO-CH_2-C\equiv CH$ | " | 4 |
| 38 | " | " | – | $-COO-CH_2$-(2-Methylphenyl) | Harz | 4 |
| 39 | " | " | – | -COO- 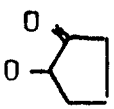 | Oel | 4 |
| 40 | " | " | – | $-COO-N=C(CH_3)_2$ | " | 2 |
| 41 | " | " | – | $CH_3$<br>$-COO-N=C$<br>$C_2H_5$ | " | 2 |
| 42 | " | " | – | $CH_3$<br>$-COO-N=C$<br>$CH(CH_3)_2$ | " | 2 |

Fortsetzung Tabelle 1

| Bsp. Nr. | $R^1$ | $R^2$ | $R^3$ | -X | Fp. [°C] | hergestellt nach Beispiel |
|---|---|---|---|---|---|---|
| 43 | $C_2H_5$ | $C_2H_5$ | — | $-COO-N=C(C_2H_5)_2$ | Oel | 2 |
| 44 | " | " | — | 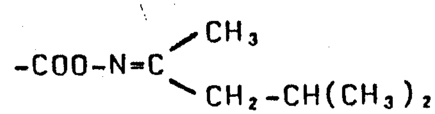 | " | 2 |
| 45 | " | " | — | 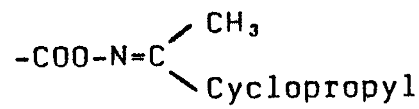 | Oel | 2 |
| 46 | " | " | — | 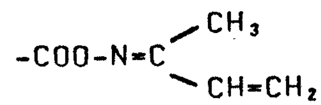 | " | 2 |
| 47 | " | " | — | 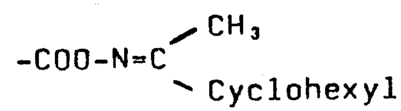 | " | 2 |
| 48 | " | " | — | 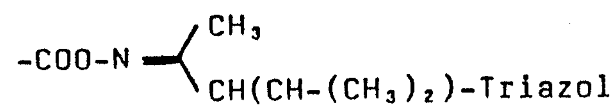 | " | 2 |
| 49 | " | " | — | 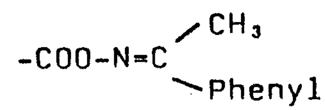 | " | 2 |
| 50 | " | " | — | 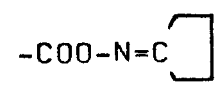 | Harz | 2 |

Fortsetzung Tabelle 1

| Bsp. Nr. | $R^1$ | $R^2$ | $R^3$ | -X | Fp. [°C] | hergestellt nach Beispiel |
|---|---|---|---|---|---|---|
| 51 | $C_2H_5$ | $C_2H_5$ | - | $-COO-N=C$ 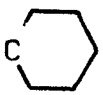 | Harz | 2 |
| 52 | " | " | - | $-COO-N(C_2H_5)_2$ | | 2 |
| 53 | " | " | - | $-COS-CH_2-COOH$ | | 2 |
| 54 | " | " | - | $-COS-CH_2-COOC_2H_5$ | Oel | 2 |
| 55 | " | " | - | $-CONH-C(CH_3)_2-CH_2-OH$ | | 3 |
| 56 | " | " | - | $-CONH-CH_2-$Phenyl | | 3 |
| 57 | " | " | - | $-CONH-CH_2-CN$ | 128-34 | 3 |
| 58 | " | " | - | $CH_3$<br>\|<br>$-CONH-C-CH(CH_3)_2$<br>\|<br>$C \equiv N$ | Harz | 3 |
| 59 | " | " | - | $-CONH-C(CH_3)-CONH_2$<br>\|<br>$CH(CH_3)_2$ | | 3 |
| 60 | " | " | - | $-CONH-CH_2-COOH$ | 210-4 | - 3) |
| 61 | " | " | - | $-CONH-CH_2-COOCH_3$ | 155-9 | 3 |
| 62 | " | " | - | $-CONH-CH_2-COOC_2H_5$ | 140-1 | 3 |
| 63 | " | " | - | $-CONH-CH_2-CHO$ | 115-21 | - 4) |

Fortsetzung Tabelle 1

| Bsp. Nr. | $R^1$ | $R^2$ | $R^3$ | -X | Fp. [°C] | hergestellt nach Beispiel |
|---|---|---|---|---|---|---|
| 64 | $C_2H_5$ | $C_2H_5$ | — | -CONH-(3,4-di-Cl-Phenyl) | 220 (Zers.) | 3 |
| 65 | " | " | — | $-CONH-NH_2$ | 145-7 | 3 |
| 66 | " | " | — | $-CONH-N(CH_3)_2$ | 123-8 | 3 |
| 67 | " | " | — | -CONH-NHCHO | Harz | 3 |
| 68 | " | " | — | -CONH-OH | Harz | 3 |
| 69 | " | " | — | $-CONH-O-CH_2-$Phenyl | 143-5 | 3 |
| 70 | " | " | — | $-CONH-NH-CONH_2$ | Oel | 3 |
| 71 | " | " | — | $-CONH-NH-CSNH_2$ | 148 (Zers.) | 3 |
| 72 | " | " | — | $-CONH-SO_2-$(4-Methylphenyl) | 143-7 | 3 |
| 73 | " | " | — | 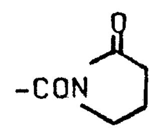 | Oel | 3 |
| 74 | " | " | — | 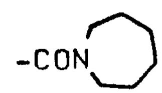 | Harz | 3 |
| 75 | " | " | — | 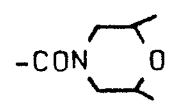 | Oel | 3 |
| 76 | " | " | — | 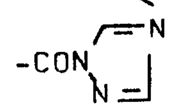 | " | 3 |

Fortsetzung Tabelle 1

| Bsp. Nr. | $R^1$ | $R^2$ | $R^3$ | -X | Fp. [°C] | hergestellt nach Beispiel |
|---|---|---|---|---|---|---|
| 77 | $C_2H_5$ | $C_2H_5$ | – | 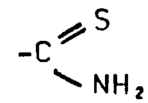 | | 3 |
| 78 | " | " | – | 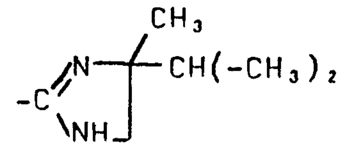 | | 11 |
| 79 | " | " | – | 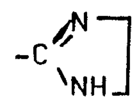 | | 11 |
| 80 | " | " | – | 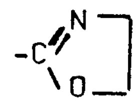 | | 6 |
| 81 | " | " | – | 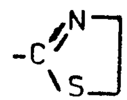 | | 6 |
| 82 | " | " | – | 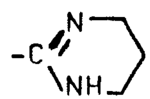 | | 11 |
| 83 | " | " | – | 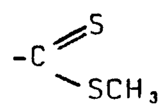 | | – 2) |

Fortsetzung Tabelle 1

| Bsp. Nr. | $R^1$ | $R^2$ | $R^3$ | -X | Fp. [°C] | hergestellt nach Beispiel |
|---|---|---|---|---|---|---|
| 84 | $C_2H_5$ | $CH(CH_3)_2$ | – | -CONH-Cyclohexyl | | 3 |
| 85 | " | " | – | 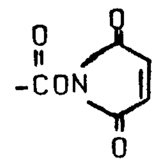 | | 2 |
| 86 | $CH(CH_3)_2$ | " | – | $-C(=S)SH$ | | - 5) |
| 87 | " | " | – | $-C(=S)S^{\ominus}Na^{\oplus}$ | | - 5) |
| 88 | " | " | – | $-C(=S)S^{\ominus}NH_4^{\oplus}$ | | - 5) |
| 89 | " | " | – | $-C(=S)S^{\ominus}\cdot NH_2^{\oplus}(CH_3)_2$ | | - 5) |
| 90 | " | " | – | 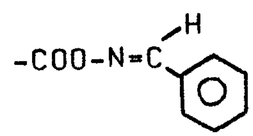 | | 2 |
| 91 | " | " | – | $-COO-CH_2-COO^{\ominus}NH_4^{\oplus}$ | | 2 |

Fortsetzung Tabelle 1

| Bsp. Nr. | $R^1$ | $R^2$ | $R^3$ | -X | Fp. [°C] | hergestellt nach Beispiel |
|---|---|---|---|---|---|---|
| 92 | $C_2H_5$ | $C_2H_5$ | - | $-CH_2-O-COCH_3$ | Oel | - 6) |
| 93 | " | " | - | $-CH_2-O-CONHCH_3$ | 113-5 | - 6) |
| 94 | " | " | - | $-CH_2-O-CO-$Phenyl | Oel | - 6) |
| 95 | " | " | - | $-CH_2-O-CH_2-$(2-Methylphenyl) | " | - 6) |
| 96 | " | " | - | 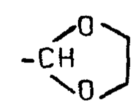 | | - 7) |
| 97 | " | " | - | $-CH_2-O-CO-CH($4-Chlorphenyl$)$ with $CH(CH_3)_2$ on CH | Oel | - 6) |
| 98 | " | " | - | 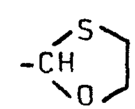 | | - 7) |

Fortsetzung Tabelle 1

| Bsp. Nr. | $R^1$ | $R^2$ | $R^3$ | X | Fp [°C] | hergest. n. Bsp. Nr. |
|---|---|---|---|---|---|---|
| 99 | $C_2H_5$ | $C_2H_5$ | - | $-C(=S)OC_2H_5$ | Oel | - 2) |
| 100 | " | " | - | $-COO-CH_2-C(=O)-CH_3$ | " | 4 |
| 101 | " | " | - | $-COO-CH_2-CH(OH)-CH_3$ | " | - 8) |
| 102 | " | " | - | $-CONH-SO_2-(4-CH_3)$-Phenyl | 143-7 | 10 |
| 103 | " | " | - | $-CONH-SO_2-(2-Cl)$-Phenyl | | 10 |
| 104 | " | " | - | $-COO-CH_2-COOH\cdot N(-CH_2-CH_2-OH)_3$ | 88-90 | - 9) |
| 105 | " | " | - | $-COO-N=C(CH_3)-C_3H_5$ (Sulfat) | Harz | - 10) |
| 106 | " | " | - | 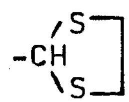 | Oel | - 7) |
| 107 | " | " | - | $-CH(SC_2H_5)_2$ | 86-9 | - 7) |
| 108 | " | " | - | $-C(CH_3)=NOH$ | 143-6 | 14 |
| 109 | " | " | - | $-C(CH_3)=NOCOCH_3$ | 91-5 | - 6) |
| 110 | " | " | - | $-C(NH_2)=N-OCH_3$ | Oel | - 6) |
| 111 | " | " | - | $-C(NH_2)=N-OC_2H_5$ | " | - 6) |
| 112 | " | $CH_3$ | - | $-CH_2OH$ | 61-3 | 12 |
| 113 | " | " | - | $-CHO$ | Oel | 13 |
| 114 | " | " | - | $-CH=NOH$ | 168-74 | 14 |

Fortsetzung Tabelle 1

| Bsp. Nr. | $R^1$ | $R^2$ | $R^3$ | X | Fp [°C] | hergest. n. Bsp. Nr. |
|---|---|---|---|---|---|---|
| 115 | $C_2H_5$ | $C_2H_5$ | - | 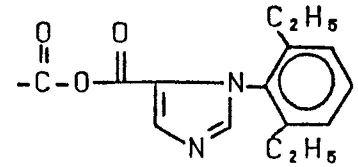 | 120-8 | _11) |
| 116 | " | " | - | $-C(O)-O-C(O)-C(CH_3)_2$ | 117-20 | _11) |
| 117 | " | " | - | 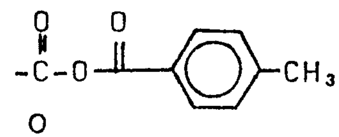 | Harz | _11) |
| 118 | " | " | - | $-C(O)-N=C=S$ | Öl | _12) |

Fußnoten zu Tabelle 1

1) die beiden anderen Reste $R^3$ stehen für Wasserstoff

2) hergestellt durch Umsetzung des Ester/Thioesters mit $P_4S_{10}$

3) hergestellt durch alkalische Verseifung des Esters in Beispiel 61

4) hergestellt durch saure Spaltung des Dimethylacetals im Anschluß an die Amidbildung

5) hergestellt aus dem Aldehyd durch Umsetzung mit Ammoniumsulfid und ggf. anschließende Salzbildung

6) hergestellt durch Alkylierung, Acylierung oder Carbamylierung der OH-Verbindung

7) hergestellt durch Acetalisierung des Aldehyds

8) hergestellt durch Natriumborhydridreduktion des Ketons von Beispiel 100

9) hergestellt durch a) Hydrierung des Benzylesters zur Säure, b) Salzbildung mit dem Amin

10) hergestellt durch Umsetzung des Imidazols mit Schwefelsäure

11) hergestellt durch Umsetzung der entsprechenden Säurechloride mit dem Natriumsalz X = COONa

12) hergestellt durch Umsetzung des entsprechenden Säurechlorids mit Bleirhodanid

...

0185961

## Biologische Beispiele

### 1. Wuchshemmung an Getreide

In Schalenversuchen im Gewächshaus werden junge Getreidepflanzen (Weizen, Gerste, Roggen) im 3-Blattstadium mit erfindungsgemäßen Verbindungen in verschiedenen Wirkstoffkonzentrationen (kg/ha) tropfnaß gespritzt.

Nachdem die unbehandelten Kontrollpflanzen eine Wuchshöhe von etwa 55 cm erreicht haben, wird bei allen Plfanzen der Zuwachs gemessen und die Wuchshemmung in % des Zuwachses der Kontrollpflanzen berechnet. Es wird außerdem die phytotoxische Wirkung der Verbindung beobachtet. Die Wuchshemmung wird als prozentualer Wert ermittelt, wobei 100% den Stillstand des Wachstums und 0% ein Wachstum entsprechend den unbehandelten Kontrollpflanzen bedeuten. Es zeigt sich, daß die Verbindungen sehr gute wachstumsregulierende Eigenschaften besitzen, siehe Tabelle I.

Tabelle I

| Verbindung nach Beispiel Nr. | Anwendungskonzentr. (kg/ha) | Wuchsshemmung in % bei | | | Phytotox. Wirkung |
|---|---|---|---|---|---|
| | | Weizen | Gerste | Roggen | |
| 2 | 2,5 | 23 | 37 | 27 | keine |
| | 1,25 | 12 | 18 | 21 | Schäden |
| 3 | 2,5 | 22 | 35 | 26 | keine |
| | 1,25 | 13 | 17 | 20 | Schäden |
| 4 | 2,5 | 21 | 34 | 26 | keine |
| | 1,25 | 14 | 17 | 20 | Schäden |
| 5 | 2,5 | 18 | 30 | 21 | keine |
| | 1,25 | 9 | 13 | 9 | Schäden |
| 6 | 2,5 | 17 | 25 | 17 | keine |
| | 1,25 | 8 | 13 | 9 | Schäden |

0185961

Tabelle I, Forts.

| Verbindung nach Beispiel Nr. | Anwendungskonzentr. (kg/ha) | Wuchsshemmung in % bei Weizen | Gerste | Roggen | Phytotox. Wirkung |
|---|---|---|---|---|---|
| 7 | 2,5 | 24 | 39 | 28 | keine |
| | 1,25 | 15 | 19 | 21 | Schäden |
| 8 | 2,5 | 22 | 34 | 25 | keine |
| | 1,25 | 12 | 18 | 17 | Schäden |
| 12 | 2,5 | 23 | 33 | 24 | keine |
| | 1,25 | 11 | 17 | 17 | Schäden |
| 13 | 2,5 | 24 | 37 | 27 | keine |
| | 1,25 | 15 | 19 | 19 | Schäden |
| 14 | 2,5 | 19 | 32 | 24 | keine |
| | 1,25 | 11 | 16 | 13 | Schäden |
| 36 | 2,5 | 19 | 33 | 23 | keine |
| | 1,25 | 10 | 17 | 13 | Schäden |
| 37 | 2,5 | 15 | 25 | 19 | keine |
| | 1,25 | 8 | 12 | 7 | Schäden |
| 40 | 2,5 | 25 | 38 | 27 | keine |
| | 1,25 | 16 | 19 | 21 | Schäden |
| 42 | 2,5 | 21 | 31 | 19 | keine |
| | 1,25 | 12 | 16 | 14 | Schäden |
| 45 | 2,5 | 19 | 32 | 22 | keine |
| | 1,25 | 11 | 15 | 15 | Schäden |
| 48 | 2,5 | 22 | 34 | 26 | keine |
| | 1,25 | 13 | 17 | 20 | Schäden |
| 51 | 2,5 | 25 | 37 | 27 | keine |
| | 1,25 | 16 | 18 | 20 | Schäden |
| 54 | 2,5 | 22 | 33 | 24 | keine |
| | 1,25 | 13 | 19 | 18 | Schäden |
| 57 | 2,5 | 23 | 31 | 23 | keine |
| | 1,25 | 12 | 18 | 17 | Schäden |
| 60 | 2,5 | 23 | 32 | 24 | keine |
| | 1,25 | 13 | 19 | 17 | Schäden |
| 61 | 2,5 | 23 | 31 | 25 | keine |
| | 1,25 | 13 | 19 | 18 | Schäden |

Tabelle I

| Verbindung nach Beispiel Nr. | Anwendungskonzentr. (kg/ha) | Wuchsshemmung in % bei | | | Phytotox. Wirkung |
|---|---|---|---|---|---|
| | | Weizen | Gerste | Roggen | |
| 65 | 2,5 | 21 | 29 | 24 | keine |
| | 1,25 | 14 | 19 | 18 | Schäden |
| 66 | 2,5 | 22 | 31 | 25 | keine |
| | 1,25 | 14 | 19 | 18 | Schäden |
| 68 | 2,5 | 24 | 34 | 27 | keine |
| | 1,25 | 20 | 21 | 21 | Schäden |
| 70 | 2,5 | 22 | 27 | 25 | keine |
| | 1,25 | 12 | 18 | 21 | Schäden |
| 73 | 2,5 | 21 | 28 | 24 | keine |
| | 1,25 | 12 | 17 | 16 | Schäden |
| 76 | 2,5 | 21 | 27 | 23 | keine |
| | 1,25 | 13 | 17 | 15 | Schäden |
| 95 | 2,5 | 19 | 25 | 22 | keine |
| | 1,25 | 14 | 17 | 15 | Schäden |
| 103 | 2,5 | 23 | 29 | 27 | keine |
| | 1,25 | 16 | 19 | 16 | Schäden |
| 105 | 2,5 | 19 | 25 | 25 | keine |
| | 1,25 | 15 | 18 | 16 | Schäden |
| 115 | 2,5 | 24 | 38 | 31 | keine |
| | 1,25 | 14 | 19 | 24 | Schäden |
| A | 2,5 | 27 | 8 | 10 | keine |
| | 1,25 | 23 | 0 | 0 | Schäden |

A = 2-Chlorethyltriethylammoniumchlorid

## 2. Wuchshemmung in Wasserreis

Reispflanzen werden in Kleinparzellen (2 m x 2 m) angezogen und im Stadium der maximalen Bestockung mit erfindungsgemäßen Verbindungen behandelt. Die Substanzen werden sowohl durch Spritzung appliziert als auch in das Wasser gegeben.

0185961

3 Wochen nach der Behandlung wird bei allen Pflanzen der Zuwachs gemessen und die Wuchshemmung in % des Zuwachses der Kontrollpflanzen berechnet. Es wird außerdem auf eine mögliche phytotoxische Wirkung der Verbindungen geachtet.

Die Wuchshemmung wird als prozentualer Wert ermittelt, wobei 100% den Stillstand des Wachstums und 0% ein Wachstum entsprechend dem der unbehandelten Kontrollpflanzen bedeuten. Es zeigt sich die sehr gute Wuchshemmung der Verbindungen, siehe Tabelle II.

Tabelle II

| Verbindung nach Beispiel Nr. | Anwendungskonzentr. (kg/ha) | Wuchsshemmung (%) | Phytotox. Wirkung |
|---|---|---|---|
| 2 | 1,25 | 22 | keine |
| | 0,62 | 25 | Schäden |
| 3 | 1,25 | 21 | keine |
| | 0,62 | 16 | Schäden |
| 4 | 1,25 | 21 | keine |
| | 0,62 | 16 | Schäden |
| 5 | 1,25 | 19 | keine |
| | 0,62 | 11 | Schäden |
| 6 | 1,25 | 18 | keine |
| | 0,62 | 11 | Schäden |
| 7 | 1,25 | 24 | keine |
| | 0,62 | 15 | Schäden |
| 8 | 1,25 | 22 | keine |
| | 0,62 | 12 | Schäden |
| 12 | 1,25 | 21 | keine |
| | 0,62 | 13 | Schäden |
| 13 | 1,25 | 24 | keine |
| | 0,62 | 16 | Schäden |
| 14 | 1,25 | 21 | keine |
| | 0,62 | 13 | Schäden |

Tabelle II, Forts.

| Verbindung nach Beispiel Nr. | Anwendungskonzentr. (kg/ha) | Wuchsshemmung (%) | Phytotox. Wirkung |
|---|---|---|---|
| 36 | 1,25 | 21 | keine |
| | 0,62 | 14 | Schäden |
| 37 | 1,25 | 19 | keine |
| | 0,62 | 11 | Schäden |
| 40 | 1,25 | 25 | keine |
| | 0,62 | 18 | Schäden |
| 42 | 1,25 | 19 | keine |
| | 0,62 | 14 | Schäden |
| 45 | 1,25 | 19 | keine |
| | 0,62 | 13 | Schäden |
| 48 | 1,25 | 19 | keine |
| | 0,62 | 14 | Schäden |
| 51 | 1,25 | 26 | keine |
| | 0,62 | 19 | Schäden |
| 54 | 1,25 | 21 | keine |
| | 0,62 | 14 | Schäden |
| 57 | 1,25 | 20 | keine |
| | 0,62 | 13 | Schäden |
| 60 | 1,25 | 21 | keine |
| | 0,62 | 13 | Schäden |
| 61 | 1,25 | 20 | keine |
| | 0,62 | 13 | Schäden |
| 65 | 1,25 | 21 | keine |
| | 0,62 | 14 | Schäden |
| 66 | 1,25 | 21 | keine |
| | 0,62 | 12 | Schäden |
| 68 | 1,25 | 25 | keine |
| | 0,62 | 18 | Schäden |
| 70 | 1,25 | 21 | keine |
| | 0,62 | 14 | Schäden |
| 73 | 1,25 | 22 | keine |
| | 0,62 | 14 | Schäden |

Tabelle II, Forts.

| Verbindung nach Beispiel Nr. | Anwendungskonzentr. (kg/ha) | Wuchsshemmung (%) | Phytotox. Wirkung |
|---|---|---|---|
| 76 | 1,25 | 19 | keine |
| | 0,62 | 14 | Schäden |
| 95 | 1,25 | 18 | keine |
| | 0,62 | 14 | Schäden |
| 103 | 1,25 | 21 | keine |
| | 0,62 | 15 | Schäden |
| 105 | 1,25 | 20 | keine |
| | 0,62 | 14 | Schäden |
| 115 | 1,25 | 28 | keine |
| | 0,62 | 19 | Schäden |

3. Wuchshemmung an Sojabohnen

Etwa 10 cm große Sojabohnen werden mit den Wirkstoffzubereitungen tropfnaß bespritzt. Nach 3 Wochen wird bonitiert.

Die Wuchshemmung wird als prozentualer Wert ermittelt, wobei 100% den Stillstand des Wachstums und 0% ein Wachstum entsprechend dem der unbehandelten Kontrollpflanzen bedeuten. Es zeigt sich, daß die Verbindungen sehr gute wachstumsregulierende Eigenschaften besitzen, siehe Tabelle III.

Tabelle III

| Verbindung nach Beispiel Nr. | Anwendungskonzentr. (kg/ha) | Wuchsshemmung (%) | Phytotox. Wirkung |
|---|---|---|---|
| 2 | 2,5 | 20 | keine Schäden |

Tabelle III, Forts.

| Verbindung nach Beispiel Nr. | Anwendungskonzentr. (kg/ha) | Wuchsshemmung (%) | Phytotox. Wirkung |
|---|---|---|---|
| 3 | 2,5 | 21 | keine Schäden |
| 4 | 2,5 | 21 | keine Schäden |
| 5 | 2,5 | 18 | keine Schäden |
| 6 | 2,5 | 19 | keine Schäden |
| 7 | 2,5 | 26 | keine Schäden |
| 8 | 2,5 | 21 | keine Schäden |
| 12 | 2,5 | 21 | keine Schäden |
| 13 | 2,5 | 25 | keine Schäden |
| 14 | 2,5 | 20 | keine Schäden |
| 36 | 2,5 | 19 | keine Schäden |
| 37 | 2,5 | 17 | keine Schäden |
| 40 | 2,5 | 26 | keine Schäden |
| 42 | 2,5 | 19 | keine Schäden |
| 45 | 2,5 | 18 | keine Schäden |
| 48 | 2,5 | 17 | keine Schäden |

Tabelle III, Forts.

| Verbindung nach Beispiel Nr. | Anwendungskonzentr. (kg/ha) | Wuchsshemmung (%) | Phytotox. Wirkung |
|---|---|---|---|
| 51 | 2,5 | 24 | keine Schäden |
| 54 | 2,5 | 19 | keine Schäden |
| 57 | 2,5 | 18 | keine Schäden |
| 60 | 2,5 | 21 | keine Schäden |
| 61 | 2,5 | 17 | keine Schäden |
| 65 | 2,5 | 17 | keine Schäden |
| 66 | 2,5 | 18 | keine Schäden |
| 68 | 2,5 | 26 | keine Schäden |
| 70 | 2,5 | 19 | keine Schäden |
| 73 | 2,5 | 19 | keine Schäden |
| 76 | 2,5 | 21 | keine Schäden |
| 95 | 2,5 | 20 | keine Schäden |
| 103 | 2,5 | 22 | keine Schäden |
| 105 | 2,5 | 18 | keine Schäden |
| 115 | 2,5 | 15 | keine Schäden |

Patentansprüche:

1. Verbindungen der allgemeinen Formel (I)

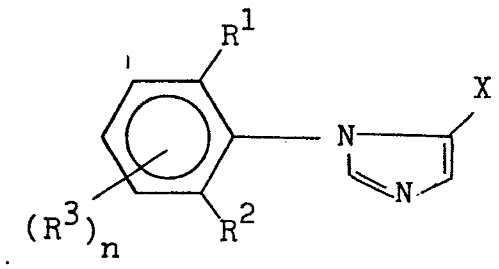

(I),

worin

$R^1$, $R^2$ unabhängig voneinander $(C_1-C_4)$Alkyl;

$R^3$ $(C_1-C_4)$Alkyl, $(C_1-C_4)$Alkoxy oder Halogen, wobei bei n 1 die Substituenten auch unterschiedlich sein können;

n 0, 1, 2 oder 3;

X -CN, -COOR$^4$, -COSR$^5$, 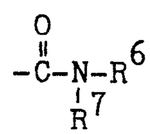, 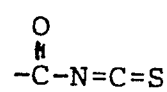,

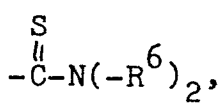, 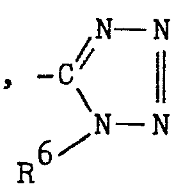, 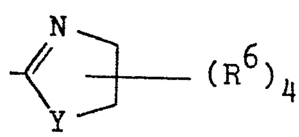,

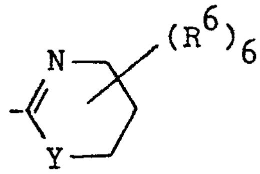, 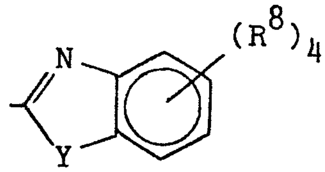,

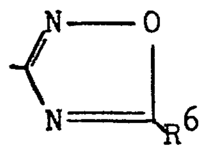 , 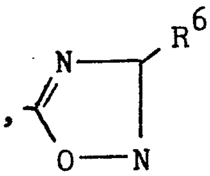 , 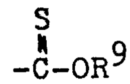 , 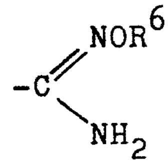

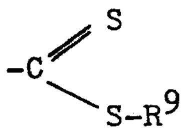 , $-CH_2-OR^{16}$ , 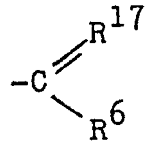 oder

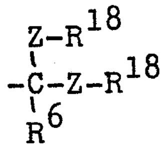 ;

Y O, S oder $N-R^6$;

Z O oder S;

$R^4$ substituiertes $(C_1-C_{12})$Alkyl, vorzugsweise $(C_1-C_6)$Alkyl, das bis zu dreifach, vorzugsweise einfach substituiert ist durch Hydroxy-$(C_1-C_4)$Alkoxy-$(C_1-C_4)$alkoxy, Cyclo$(C_3-C_7)$-alkyl, Benzyloxy, Benzyloxyethoxy, Phenyl, Halogenphenyl, Methylphenyl, Cyano, Carbamoyl, Oxiranyl, Mono$(C_1-C_6)$-alkylamino, $(C_1-C_4)$Alkylthio, Trimethylsilyl, $(C_1-C_4)$-Alkanoyl, $(C_1-C_4)$Alkoxycarbonyl, Phenoxy, Phenylthio, $-O-N=C(-CH_3)_2$, Triazolyl, Imidazolyl, Carboxyl oder Carboxylat mit einem Ammonium- oder Metallkation, vorzugsweise aus der Gruppe der Alkali- oder Erdalkalimetallkationen, oder mit einem organischen Ammonium-, Phosphonium-, Sulfonium- oder Sulfoxoniumkation; Halogen$(C_3-C_6)$alkenyl; unsubstituiertes oder durch Halogen oder $(C_1-C_4)$Alkyl substituiertes Cyclo$(C_3-C_6)$-alkyl; unsubstituiertes oder durch Halogen

oder Methyl substituiertes Cyclo($C_5$-$C_6$)-alkenyl; ($C_3$-$C_6$)-Alkinyl; 1,2-Epoxyprop-3-yl; substituiertes Phenyl, welches ein- oder zweifach substituiert ist durch Halogen, Nitro, Cyano, ($C_1$-$C_4$)Alkyl, ($C_1$-$C_4$)-Alkoxycarbonyl oder ($C_1$-$C_4$)Alkoxy; $-N{=}CR^{10}R^{11}$, $-NR^6R^{12}$

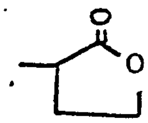 , 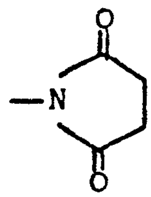 , 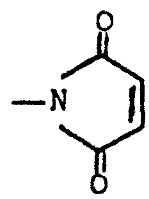 , 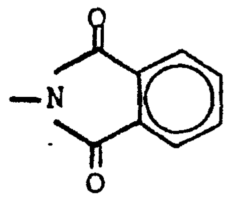 ,

(($C_1$-$C_4$)-Alkyl)carbonyl, Phenylcarbonyl, das durch Halogen , Nitro, Cyano oder ($C_1$-$C_4$)-Alkylsubstituiert sein kann, oder einen Rest der Formel

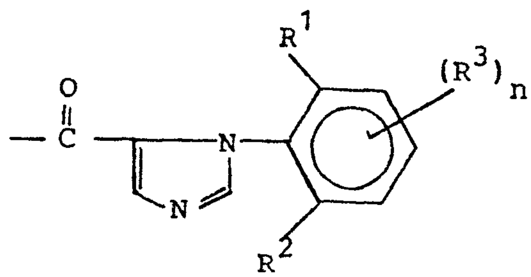

$R^5$ substituiertes ($C_1$-$C_{12}$)Alkyl, vorzugsweise ($C_1$-$C_6$)Alkyl, das bis zu zweifach, vorzugsweise einfach substituiert ist durch ($C_1$-$C_4$)Alkoxy-ethoxy, Cyclo($C_3$-$C_6$)alkyl, Benzyloxy, Phenyl, ($C_1$-$C_4$)Alkylthio, ($C_1$-$C_4$)Alkoxycarbonyl, Phenoxy, Carboxyl oder Carboxylat mit einem Ammonium- oder Metallkation, vorzugsweise aus der Gruppe der Alkali- oder Erdalkalimetallkationen, oder mit einem organischen Ammonium-, Phosphonium-, Sulfonium- oder Sulfoxoniumkation; oder Cyclo($C_3$-$C_6$)alkyl;

$R^6$ H oder $(C_1-C_4)$Alkyl;

$R^7$ substituiertes $(C_1-C_{12})$Alkyl, vorzugsweise $(C_1-C_6)$Alkyl, das bis zu zweifach, vorzugsweise einfach substituiert ist durch $(C_1-C_4)$Alkoxyethoxy, Hydroxyl, Cyclo$(C_3-C_6)$alkyl, Benzyloxy, Phenyl, Cyano, Carbamoyl, Carboxyl, $(C_1-C_4)$Alkoxycarbonyl, Formyl oder Phenoxy; Cyclo$(C_3-C_6)$alkyl; bis zu zweifach durch Halogen, Nitro, Cyano, $(C_1-C_4)$Alkyl oder $(C_1-C_4)$-Alkoxy subsituiertes Phenyl;
$-NR^6R^{13}$, $-OR^{14}$, $-NH-CO-NH_2$, $-NH-CS-NH_2$ oder $-SO_2R^{15}$;

oder $R^6$ und $R^7$ bilden - gemeinsam mit dem Stickstoffatom an das sie gebunden sind - einen gesättigten oder ungesättigten, gegebenenfalls benzokondensierten drei- bis siebengliedrigen Ring, der bis zu drei Heteroatome aus der Gruppe O,N und S enthält und der unsubstituiert oder durch $(C_1-C_4)$Alkyl oder Halogen substituiert ist und eine Oxogruppe enthalten kann;

$R^8$ unabhängig voneinander H, Halogen, $(C_1-C_4)$-Alkyl, Nitro oder Cyano;

$R^9$ H, $(C_1-C_6)$Alkyl oder Phenyl, oder bei Thiocarboxylaten ein Alkali- oder Erdalkalimetallkation, Ammonium- oder organisches Ammoniumkation;

$R^{10}$, $R^{11}$ unabhängig voneinander H, unsubstituiertes oder durch Triazolyl oder Imidazolyl substituiertes $(C_1-C_6)$Alkyl; Cyclo$(C_3-C_6)$-alkyl, $(C_3-C_6)$Alkenyl, Phenyl oder Benzyl;

oder sie bilden gemeinsam mit dem Kohlenstoffatom, an das sie gebunden sind, ein unsubstituierte oder durch Methyl oder Halogen substituiertes Cyclo($C_5$-$C_7$)alkyl;

$R^{12}$ ($C_1$-$C_4$)Alkyl, Phenyl, ($C_1$-$C_6$)Alkylcarbonyl, Benzyl, Benzoyl, Halogenbenzyl, Halogenbenzoyl oder Methylbenzoyl;

$R^{13}$ H, ($C_1$-$C_4$)Alkyl, Formyl, ($C_1$-$C_6$)Alkylcarbonyl, Benzoyl, Halogenbenzoyl, Methylbenzoyl oder Trihalogenacetyl;

$R^{14}$ H, ($C_1$-$C_4$)Alkyl oder Benzyl;

$R^{15}$ ($C_1$-$C_4$)Alkyl, Phenyl, Chlorphenyl oder Methylphenyl,

$R^{16}$ H, unsubstituiertes oder durch Phenyl oder ($C_1$-$C_4$)-Alkylphenyl, substituiertes ($C_1$-$C_{12}$)-alkyl, ($C_1$-$C_6$)Alkylcarbonyl, Halogen($C_1$-$C_6$)-alkylcarbonyl, N-($C_1$-$C_6$)Alkylcarbamoyl, Benzoyl, Halogenbenzoyl oder Methylbenzoyl;

$R^{17}$ O (einschließlich der Bisulfidaddukte) oder $=N-O-R^{16}$; und

$R^{18}$ unabhängig voneinander unsubstituiertes oder durch Phenyl, Cyclo($C_5$-$C_7$)alkyl, ($C_1$-$C_4$)Alkoxy, ($C_1$-$C_4$)Alkylthio oder Halogen substituiertes ($C_1$-$C_6$)Alkyl;
oder beide Reste $R^{18}$ bilden, gemeinsam mit Z und dem Kohlenstoffatom an das sie gebunden sind, einen unsubstituierten oder durch ($C_1$-$C_4$)Alkyl, Hydroxy-($C_1$-$C_4$)alkyl, Halogen-($C_1$-$C_4$)alkyl oder Phenyl substituierten 5- oder 6-gliedrigen Ring

bedeuten sowie deren für die Landwirtschaft einsetzbaren Salze oder Quaternisierungsprodukte.

2. Verfahren zur Herstellung der Verbindungen der allgemeinen Formel (I) nach Anspruch 1, sowie deren Salze und Quaternisierungsprodukte, dadurch gekennzeichnet, daß man Verbindungen der allgemeinen Formel (I) mit der Bedeutung von X=-COOH, $-CONH_2$, -CO-O-($C_1$-$C_4$)Alkyl oder ihre aktivierten Derivate wie die Carbonsäurehalogenide zu den Verbindungen der allgemeinen Formel (I) mit der dort definierten Bedeutung von X derivatisiert oder sie in ihre Salze und Quaternisierungsprodukte überführt.

3. Das Pflanzenwachstum regulierende Mittel mit einem wirksamen Gehalt an mindestens einer Verbindung nach Anspruch 1.

4. Verwendung von Verbindungen nach Anspruch 1 als Wachstumsregulatoren für Pflanzen.

5. Verfahren zur Wachstumsregulierung von Pflanzen, dadurch gekennzeichnet, daß man auf die Pflanzen oder die Anbaufläche eine wirksame Menge mindestens einer Verbindung nach Anspruch 1 appliziert.

Patentansprüche Österreich:

1. Pflanzenwachstumsregulierende Mittel, dadurch gekennzeichnet, daß sie eine Verbindung der allgemeinen Formel I

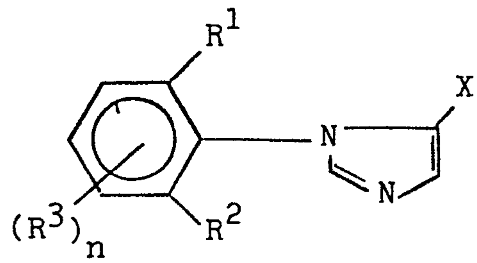

(I),

worin

$R^1$, $R^2$ unabhängig voneinander $(C_1-C_4)$Alkyl;

$R^3$ $(C_1-C_4)$Alkyl, $(C_1-C_4)$Alkoxy oder Halogen, wobei bei n 1 die Substituenten auch unterschiedlich sein können;

n 0, 1, 2 oder 3;

X -CN, $-COOR^4$, $-COSR^5$, 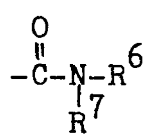, 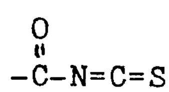

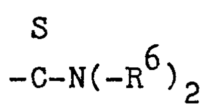, 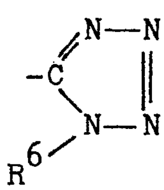, 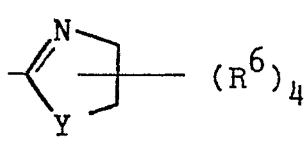

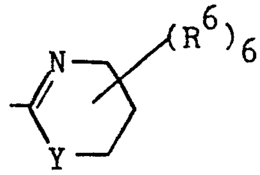, 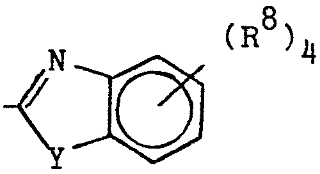,

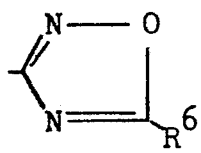 , 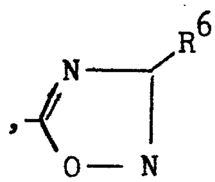 , 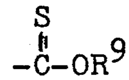 , 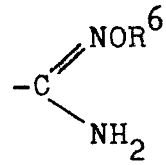

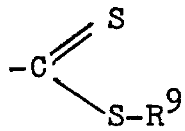 , $-CH_2-OR^{16}$ , 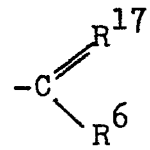 oder

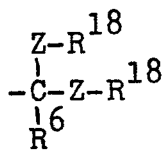 ;

Y O, S oder $N-R^6$;

Z O oder S;

$R^4$ substituiertes $(C_1-C_{12})$Alkyl, vorzugsweise $(C_1-C_6)$Alkyl, das bis zu dreifach, vorzugsweise einfach substituiert ist durch Hydroxy-$(C_1-C_4)$Alkoxy-$(C_1-C_4)$alkoxy, Cyclo$(C_3-C_7)$-alkyl, Benzyloxy, Benzyloxyethoxy, Phenyl, Halogenphenyl, Methylphenyl, Cyano, Carbamoyl, Oxiranyl, Mono$(C_1-C_6)$-alkylamino, $(C_1-C_4)$Alkylthio, Trimethylsilyl, $(C_1-C_4)$-Alkanoyl, $(C_1-C_4)$Alkoxycarbonyl, Phenoxy, Phenylthio, $-O-N=C(-CH_3)_2$, Triazolyl, Imidazolyl, Carboxyl oder Carboxylat mit einem Ammonium- oder Metallkation, vorzugsweise aus der Gruppe der Alkali- oder Erdalkalimetallkationen, oder mit einem organischen Ammonium-, Phosphonium-, Sulfonium- oder Sulfoxoniumkation; Halogen$(C_3-C_6)$alkenyl; unsubstituiertes oder durch Halogen oder $(C_1-C_4)$Alkyl substituiertes Cyclo$(C_3-C_6)$-alkyl; unsubstituiertes oder durch Halogen

oder Methyl substituiertes Cyclo($C_5$-$C_6$)-alkenyl;($C_3$-$C_6$)-Alkinyl; 1,2-Epoxyprop-3-yl; substituiertes Phenyl, welches ein- oder zweifach substituiert ist durch Halogen, Nitro, Cyano, ($C_1$-$C_4$)Alkyl, ($C_1$-$C_4$)-Alkoxycarbonyl oder ($C_1$-$C_4$)Alkoxy; $-N=CR^{10}R^{11}$, $-NR^6R^{12}$

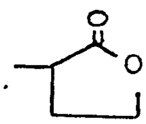 , 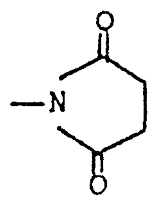 , 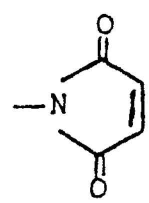 , 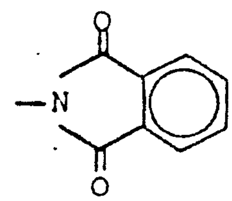 ,

(($C_1$-$C_4$)-Alkyl)carbonyl, Phenylcarbonyl, das durch Halogen , Nitro, Cyano oder ($C_1$-$C_4$)-Alkylsubstituiert sein kann, oder einen Rest der Formel

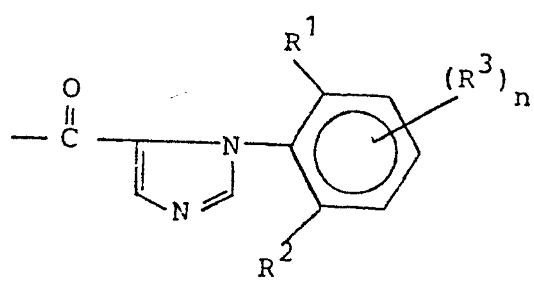

$R^5$ substituiertes ($C_1$-$C_{12}$)Alkyl, vorzugsweise ($C_1$-$C_6$)Alkyl, das bis zu zweifach, vorzugsweise einfach substituiert ist durch ($C_1$-$C_4$)Alkoxy-ethoxy, Cyclo($C_3$-$C_6$)alkyl, Benzyloxy, Phenyl, ($C_1$-$C_4$)Alkylthio, ($C_1$-$C_4$)Alkoxycarbonyl, Phenoxy, Carboxyl oder Carboxylat mit einem Ammonium- oder Metallkation, vorzugsweise aus der Gruppe der Alkali- oder Erdalkalimetallkationen, oder mit einem organischen Ammonium-, Phosphonium-, Sulfonium- oder Sulfoxoniumkation; oder Cyclo($C_3$-$C_6$)alkyl;

$R^6$ H oder $(C_1-C_4)$Alkyl;

$R^7$ substituiertes $(C_1-C_{12})$Alkyl, vorzugsweise $(C_1-C_6)$Alkyl, das bis zu zweifach, vorzugsweise einfach substituiert ist durch $(C_1-C_4)$Alkoxy-ethoxy, Hydroxyl, Cyclo$(C_3-C_6)$alkyl, Benzyloxy, Phenyl, Cyano, Carbamoyl, Carboxyl, $(C_1-C_4)$Alkoxycarbonyl, Formyl oder Phenoxy; Cyclo$(C_3-C_6)$alkyl; bis zu zweifach durch Halogen, Nitro, Cyano, $(C_1-C_4)$Alkyl oder $(C_1-C_4)$-Alkoxy subsituiertes Phenyl; $-NR^6R^{13}$, $-OR^{14}$, $-NH-CO-NH_2$, $-NH-CS-NH_2$ oder $-SO_2R^{15}$;

oder $R^6$ und $R^7$ bilden - gemeinsam mit dem Stickstoffatom an das sie gebunden sind - einen gesättigten oder ungesättigten, gegebenenfalls benzokondensierten drei- bis siebengliedrigen Ring, der bis zu drei Heteroatome aus der Gruppe O,N und S enthält und der unsubstituiert oder durch $(C_1-C_4)$Alkyl oder Halogen substituiert ist und eine Oxogruppe enthalten kann;

$R^8$ unabhängig voneinander H, Halogen, $(C_1-C_4)$-Alkyl, Nitro oder Cyano;

$R^9$ H, $(C_1-C_6)$Alkyl oder Phenyl, oder bei Thiocarboxylaten ein Alkali- oder Erdalkalimetallkation, Ammonium- oder organisches Ammoniumkation;

$R^{10}$, $R^{11}$ unabhängig voneinander H, unsubstituiertes oder durch Triazolyl oder Imidazolyl substituiertes $(C_1-C_6)$Alkyl; Cyclo$(C_3-C_6)$-alkyl, $(C_3-C_6)$Alkenyl, Phenyl oder Benzyl;

oder sie bilden gemeinsam mit dem Kohlenstoffatom, an das sie gebunden sind, ein unsubstituierte oder durch Methyl oder Halogen substituiertes Cyclo($C_5$-$C_7$)alkyl;

$R^{12}$ ($C_1$-$C_4$)Alkyl, Phenyl, ($C_1$-$C_6$)Alkylcarbonyl, Benzyl, Benzoyl, Halogenbenzyl, Halogenbenzoyl oder Methylbenzoyl;

$R^{13}$ H, ($C_1$-$C_4$)Alkyl, Formyl, ($C_1$-$C_6$)Alkylcarbonyl, Benzoyl, Halogenbenzoyl, Methylbenzoyl oder Trihalogenacetyl;

$R^{14}$ H, ($C_1$-$C_4$)Alkyl oder Benzyl;

$R^{15}$ ($C_1$-$C_4$)Alkyl, Phenyl, Chlorphenyl oder Methylphenyl,

$R^{16}$ H, unsubstituiertes oder durch Phenyl oder ($C_1$-$C_4$)-Alkylphenyl, substituiertes ($C_1$-$C_{12}$)-alkyl, ($C_1$-$C_6$)Alkylcarbonyl, Halogen($C_1$-$C_6$)-alkylcarbonyl, N-($C_1$-$C_6$)Alkylcarbamoyl, Benzoyl, Halogenbenzoyl oder Methylbenzoyl;

$R^{17}$ O (einschließlich der Bisulfidaddukte) oder =N-O-$R^{16}$; und

$R^{18}$ unabhängig voneinander unsubstituiertes oder durch Phenyl, Cyclo($C_5$-$C_7$)alkyl, ($C_1$-$C_4$)Alkoxy, ($C_1$-$C_4$)Alkylthio oder Halogen substituiertes ($C_1$-$C_6$)Alkyl;
oder beide Reste $R^{18}$ bilden, gemeinsam mit Z und dem Kohlenstoffatom an das sie gebunden sind, einen unsubstituierten oder durch ($C_1$-$C_4$)Alkyl, Hydroxy-($C_1$-$C_4$)alkyl, Halogen-($C_1$-$C_4$)alkyl oder Phenyl substituierten 5- oder 6-gliedrigen Ring

bedeuten oder deren für die Landwirtschaft einsetzbaren Salze oder Quaternisierungsprodukte enthalten.

2. Verfahren zur Herstellung der Verbindungen der allgemeinen Formel (I) nach Anspruch 1, sowie deren Salze und Quaternisierungsprodukte, dadurch gekennzeichnet, daß man Verbindungen der allgemeinen Formel (I) mit der Bedeutung von X=-COOH, $-CONH_2$, -CO-O-$(C_1-C_4)$Alkyl oder ihre aktivierten Derivate wie die Carbonsäurehalogenide zu den Verbindungen der allgemeinen Formel (I) mit der dort definierten Bedeutung von X derivatisiert oder sie in ihre Salze und Quaternisierungsprodukte überführt.

3. Verwendung von Verbindungen nach Anspruch 1 als Wachstumsregulatoren für Pflanzen.

4. Verfahren zur Wachstumsregulierung von Pflanzen, dadurch gekennzeichnet, daß man auf die Pflanzen oder die Anbaufläche eine wirksame Menge mindestens einer Verbindung nach Anspruch 1 appliziert.